# EUROPEAN PATENT APPLICATION

(11) **EP 3 065 095 A1**
(43) Date of publication of application: **07.09.2016**
(21) Application number: 16164776.3
(22) Date of filing: 06.01.2012
(51) Int. Cl.: G06Q 10/06, G06Q 50/22, G06F 19/00, A61M 5/172

(54) **SYSTEM, METHOD, AND ARTICLE TO PROMPT BEHAVIOR CHANGE**

(30) Priority: 10.01.2011 US 201161431416 P
(62) Divisional of application: 12734126.1
(71) Applicant: Proteus Digital Health, Inc., Redwood City, CA 94065 (US)
(72) Inventor: O' REILLY, David, Palo Alto, CA California 94301 (US); IONESCU, Arna Diana, San Francisco, CA California 94110 (US); DUFFY, Sean Patrick, San Francisco, CA California 94110 (US); SILVER, Jesse Amos, San Francisco, CA California 94107 (US); FETHERSTONHAUGH, David Rae, Danville, CA California 94526 (US); RICHTER, Schirin Lucie, Palo Alto, CA California 94301 (US)
(74) Representative: Keane, David Colman

(57) **Abstract**

System, method, and article to prompt behavior change are provided. Various aspects of the system include health promotion data generated and / or communicated from / to a device; a methodology module associated with software / processor to identify at least one behavior change methodology associated with the health promotion data; and an instruction module associated with a software / processor to initiate the identified at least one behavior change methodology.

## Description

### Cross Reference to Related Applications

This application claims the benefit of U.S. Provisional Patent Application No. 61/431,416 entitled "System, Method and Article to Prompt Behavior Change" and filed on January 10, 2011, which is herein entirely incorporated by reference.

### Field of the Invention

The present invention relates generally to the technical fields of health-related devices and communications. More specifically, and in various illustrative embodiments, the present invention relates to a system, method, and apparatus of promoting behavior change based on underlying health data.

### Introduction

Lack of adherence to medication and other health regimens may be a significant issue. Many individuals may fail to engage in behaviors necessary to sustain adherence to therapies. Traditional mental models such as physician instruction, long-term health rewards, and management of disease conditions simply fail to support sustained adherence to the therapies. Such mental models may be quite abstract and difficult to conceive. Patients may not identify with abstractions such as, *"If I take this pill every day for the next ten years, I may live longer."* Failure to adhere to therapies may result in unnecessary disease progression, wasted medical resources, and other untoward outcomes.

What is needed, then, is a set of motivators capable of prompting sustained behavior change, etc. that may be associated with various improved outcomes.

### Summary

The present disclosure seeks to address at least some of these problems with a system, method, and article to prompt behavior change. Various aspects include mechanisms for sustaining behavior change in consumers. Such behavior changes may have many and varied positive results, e.g., improved treatment outcomes, broad social usage, charitable benefits to others, etc. The system and method has broad applicability across consumer populations, disease states, geographical territories, and interested stakeholders.

### Brief Description of the Figures

**Figure 1** shows a schematic of a system to prompt behavior change.
**Figure 2** shows a system operation of the system to prompt behavior change in **Figure 1****.**
**Figure 2a** illustrates one conceptualization of a medmatch methodology.
**Figure 3** illustrates one conceptualization of a races within reach methodology.
**Figure 4** illustrates one conceptualization of a pick a desktop widget / avatar methodology.
**Figure 5** illustrates one conceptualization of a family responsibility methodology.
**Figure 6** illustrates one conceptualization of a virtual mansion methodology.
**Figure 7** illustrates one conceptualization of a daily hatch methodology.
**Figure 8** illustrates one conceptualization of a fitimals methodology.
**Figure 9** illustrates one conceptualization of a delightful comparators methodology.
**Figure 10** illustrates one conceptualization of an adhere to win methodology.
**Figure 11** illustrates one conceptualization of a shame game methodology.
**Figure 12** illustrates one conceptualization of a pledge matching methodology.
**Figure 13** illustrates one conceptualization of a help from my friends methodology.
**Figure 14** illustrates one conceptualization of a love buzz methodology.
**Figure 15** illustrates one conceptualization of a patch alerts methodology.
**Figure 16** illustrates one conceptualization of a done! buzz methodology.
**Figure 17** illustrates one conceptualization of a plug methodology.
**Figure 18** illustrates one conceptualization of a real patient profiles methodology.
**Figure 19** illustrates one conceptualization of a mood miner methodology.
**Figure 20** illustrates one conceptualization of a heart fit methodology.
**Figure 21** illustrates one conceptualization of a swimmer patch methodology.
**Figure 22** illustrates one conceptualization of a small steps to big results methodology.
**Figure 23** illustrates one conceptualization of a commit to healthy eating methodology.
**Figure 24** illustrates one conceptualization of a matched methodology.
**Figure 25** illustrates a method of prompting behavior change.

### Detailed Description

The present disclosure includes multiple embodiments of a system, method, and article to prompt behavior change. As described hereafter in more detail, a system and method of the present invention may be used to prompt sustainable behavior changes with various beneficial results. The results, for example, may include improved treatment outcomes, broad social usage, beneficial donations to various recipients, prudent use of medical resources, etc.

Broadly, aspects include various methodologies coupled with devices, e.g., ingestible devices, processor(s), mobile phones, computers, intelligent scales, etc., to promote behavior change.

Methodologies include, for example, schemas related to matching donations, competitive pursuits, avatar-based approaches, family-centric games, etc.

Versatile aspects provide for linkage of and / or utilization of existing automated systems with the present invention. Existing systems may include, for example, computerized matching donation systems, social networks and media, health management networks, etc.

Generally, a patient journey may follow three primary stages of (1) getting started with a therapy regimen for treatment of chronic disease(s); (2) getting into a routine to establish good habits and choices to follow and adhere to the therapy regimen and other positive lifestyle choices, e.g., diet, sleep, exercise, etc.; and (3) getting through rough spots and difficult periods in therapy and disease progression or difficulties to continue to make progress in chronic disease management and continued wellbeing. Aspects of the present invention may create product mechanisms for sustained behavior change and continuous reinforcement in each of these stages of the patient journey through the novel use of creative, automated methodologies.

One such methodology facilitates matching donations (sometimes referred to herein as "medmatch"). Under this methodology, personal act(s) of health promotion, e.g., an act of ingestion of a product such as medication or a placebo, are linked to matching altruistic goal(s). The actual confirmation of the personal act, e.g., ingestion event, is a precursor to making such a system work, as opposed to using a surrogate of the act, such as patient memory, patient reporting, smart packaging or bottles, etc.

Using this methodology, aspects of the present invention may create product mechanisms for sustained behavior change and continuous reinforcement in the following manner:
1. *Get Me Started:* medmatch activates a patient's desire to do good by giving back simply by taking the pill(s) already prescribed by their physician.
2. *Get Me Into a Routine:* medmatch gives a patient short-term reinforcement, connecting the daily and sometimes mundane task of taking a pill to a more inspirational motivation of helping another human being. Medmatch can also connect a patient with others like them, showing how the collective pill ingestions of other patients in the medmatch program are supporting a cause such as ending tuberculosis in India or providing medications to underprivileged children in a neighboring city.
3. *Get Me Through the Rough Spots:* When a patient has had setbacks in their treatment, side effects from medications, or is feeling down and frustrated about their condition, medmatch provides a higher purpose and motivation to keep going and get back on track, and to not break from the positive routine.

In the medmatch methodology, an important social effect (support for an individual or cause in need) and economic consequence (another product of real financial value being donated) is created by a personal consumer decision, and therefore the "donation transaction" must be verified and quantifiable. Direct measurement of the actual act, e.g., actual ingestion, is part of the product concept. Direct measurement may be accomplished via a variety of devices, as hereinafter discussed.

To illustrate, any individual or group ingests a medication having an integrated, ingestible device that marks an ingestion event. (Examples of such devices are described, for example, in U.S. Patent Application No. 12/564,017 filed September 21, 2009 entitled "COMMUNICATION SYSTEM WITH PARTIAL POWER SOURCE, *infra.)* The integrated device communicates relevant data, e.g., health promotion data, identification of an individual, type of medication, time of ingestion, and dosage information, to a computer or mobile device having a processor and specific software. The software / processor process the health promotion data and, upon identification of a predetermined behavior change methodology, the software / processor generate a corresponding instruction to initiate software associated with the identified behavior change methodology.

In one example, the health promotion data identify the individual who has ingested the medication. The software / processor checks a database or other storage media to determine if the identified individual has a corresponding behavior change methodology program in place. Upon determining that such a program is in place, e.g., the individual has selected "medmatch", the software / processor generates an instruction, e.g., initiates a software program, which facilitates donation of a predetermined nature to particular cause(s).

One such donation, for example, may be provision of a "matching" dose of medication to an individual in need. Thus, upon ingestion of a product by an individual, a "one for one" or "one for many" donation of a second designated product (or multiple products) to another individual, group or cause occurs.

For example, an HIV+ patient living in Boston, MA, USA may set up medmatch to donate HIV medication to another HIV+ patient living in Nairobi, Kenya every time the Boston patient took his own medication as prescribed. Rather than taking the medication because they are "told to" by their physician or because of a personal sense of longer-term health reward or disease progression fear (mental models which the scientific literature on adherence confirms have failed to support sustained adherence and persistence to drug therapy in the majority of patients living with chronic disease), the medmatch methodology supports and reinforces the act of the Boston patient taking a daily pill by creating a short-term, inspirational goal of helping another individual in need.

The methodology replaces a difficult to conceive *abstraction* (if I take this pill every day for the next 10 years I will live longer) with a *certainty* (if I take this pill today I will help someone else today). Over time, this positive daily reinforcement may be complemented by a negative reinforcement as well, in that any personal failure to take the medication as prescribed will result in the removal of the support the Boston patient has been giving to the Nairobi patient. This negative reinforcement further reinforces and sustains the Boston patient's product usage and adherence.

Medmatch may further allow product manufacturers to associate the persistent and increased usage of their products by consumers with positive social causes that the manufacturers already pursue as part of corporate responsibility, charitable giving, and global health programs. For example, the pharmaceutical company that manufactures the HIV medication for the Boston patient may link medmatch to its global AIDS program and its work with the Gates Foundation, Clinton Global Initiative, WHO and other government-sponsored program to make its expensive HIV medications available to patients in underserved places, and link the medication supplies it *already* commits for such programs to the ingestion events of individuals like its Boston consumer. This creates a reinforcing, virtuous circle, where the Boston consumer increases and sustains her adherence to the pharmaceutical company's HIV medication, the pharmaceutical company attains further recognition for its global HIV medication access program and credit for it *in its primary markets,* and the consumer and pharmaceutical company create a new, positive relationship with each other based on the new HIV pill/medmatch brand, a relationship that may be durable beyond such things as the expiration of a patent on the HIV pill itself.

Further, the nature of the consumer / pharmaceutical company relationship based on the medmatch methodology is unique and valuable-the consumer may now perceive herself to be directing and controlling the pharmaceutical company, versus a current perception born of direct-to-consumer (DTC) advertising where the consumer may feel manipulated, overcharged, and controlled by the pharmaceutical company.

Additionally, medmatch may allow companies to have a global health strategy far in advance of when they are capable of having a truly global health infrastructure to support this strategy. Small companies such as assignee Proteus Biomedical, Inc., Redwood City, CA, USA, may have a viable presence and contribution to global health needs in countries like Kenya, China and India even before such companies are able to have commercial operations in those locations.

Still further, medmatch may facilitate feeding donations back into the donor's own community, such as church groups or professional organizations, e.g., truck drivers helping each other.

Medmatch may further allow consumers and product manufacturers to create online social communities based on the linkage between personal ingestion decisions and medication donations and social causes. Organizing individual participants and recipients into groups may boost motivation because organization increases the perceived impact of an individual's actions. For example, a website may show both the Boston patient's personal impact and the cumulative good a community of other HIV+ patients in the medmatch program has done. Such a social community may have a further reinforcing effect on the individuals like the Boston patient. Through this group dynamic, support and performance becomes another driver for behavior change to adherence, and the cumulative effect that a large community can have on a cause becomes inspirational over and above what any individual can do themselves.

Medmatch need not associate an individual with a single cause related to their own medical condition, or even with health-related causes at all: product manufactures or consumers themselves will create multiple causes and social issues that an individual can select to match to their personal ingestions. An individual, for example, may decide to allocate a portion of their donations to one cause (HIV in Kenya, where each ingestion, for example, donates one pill) and a portion to another cause (childhood literacy in San Francisco, for example, where every 100 ingestions by the online cause community donates one book). A skilled artisan will note that the number of causes and the uses of medmatch to enable those causes will be many and varied to create meaning and motivation for the individuals and their online social groups, such as (a) if the patient meets their step-counting goals a pair of shoes is donated to someone in need or a donation is made toward helping those who lost limbs due to landmines; (b) if a patient meets their sleep duration goals a donation is made to a housing project that will provide allow someone in need a safe place to sleep; (c) if a diabetic patient meets their nutritional or weight loss goals food may be donated to people in need ("every pound lost is a pound of food donated"); and (d) other such linkages.

Medmatch may further extend the online social community to link the donating individual or group to the individuals or groups receiving the donations. In this aspect, motivation for the patient is created by the ability of the patient and the donation recipient to share stories and observations about their respective diseases, situations and lives. This social connection may be static, e.g., the Boston patient is able to read a biography and life story about the Nairobi patient, as well dynamic and interactive, e.g., the Boston and Nairobi patients have an ongoing dialogue with updates via various social media and networks such as Twitter, Facebook, etc.

Medmatch may further allow consumers who are patients but are not on a device-enabled medication, or are not even patients at all, to participate. In various aspects, the system and / or method may include a device-enabled placebo or vitamin tablet that can be ingested by any individual under various circumstances: (1) ingested by itself; (2) co-ingested with medications that are not device-enabled to mark ingestion by a patient; (3) ingested at any time by a patient or any other consumer, etc.

For example, the Boston patient's physician switches him to a different HIV medication because of side effects and viral resistance issues, and that medication is not device-enabled to mark ingestion. The Boston patient feels a strong connection to the medmatch program, and has come to believe in the social good his adherence is creating. He is pleased when his pharmacist tells him that there is even a medmatch tablet without a medication, e.g., a device-enabled placebo, which enables him to continue his participation in the Nairobi donation program. The pharmaceutical company that manufactured his old medication is also pleased because they are able to continue their relationship with the Boston patient while they develop or acquire other device-enabled medications for their portfolio that will be suitable for him over time.

Another example is a non-patient consumer or advocacy group interested in using medmatch for a fundraising or support event. A group raising money for a cancer institute, for example, may link its annual cycling ride charity fundraiser to medmatch, where participants all train in the months leading up to the ride while using the system, and finish use once the race is completed. In this example, the fundraising ride of 2,000 individuals may have daily training activity and placebo ingestions all linked to the matching donor giving that raises money for the charity. In a third example, a family care giver shows her support for a loved one living with a chronic illness by "matching" their drug adherence by taking a device-enabled placebo at the same times during the day that the loved one needs to take their medications. This aspect creates family support and also links family efforts to the medmatch cause that they have selected, so that the family group can see their cumulative support for their cause.

Further, product manufacturers may be able to enter into a greater number of more favorable arrangements with payors by including an altruistic element to their product marketing programs. The altruism may be applied to other members of the payor population, e.g. if a member ingests per prescribing guidelines, another member may get help with co-pays or the payor itself gets a discount.

In various aspects, the system and method have design provisions that protect against and / or take predetermined protective action(s) upon the occurrence of various events, e.g., overuse by an individual; a possible situation where someone may try to do "more good" by taking more than a prescribed amount of their medication; sudden stoppage of medication usage by the donor resulting in stoppage of the donation medication to the donee, etc.

In one example of a protective action, the donation match will only occur if the individual takes the prescribed dose, no more and no less. In various aspects, this check may be performed in an automated fashion by software / processor by comparing stored data indicating the dosage amount and frequency of a prescribed medication against health promotion data communicated by a device associated with the prescribed medication, e.g., a device-enabled pill, syringe, inhaler, etc. that communicates dose, time of dose, type of dose at each delivery event. In another example, an independent third party may manage the medmatch donation program on behalf of a product manufacturer. In still another example, the system / method automatically monitor stoppage events and ensure predetermined actions are taken, e.g., the donee's medication supply continues uninterrupted on behalf of the pharmaceutical company, the donee is matched to a new donor, the donee's physician is notified by email, instant message, etc.

### System

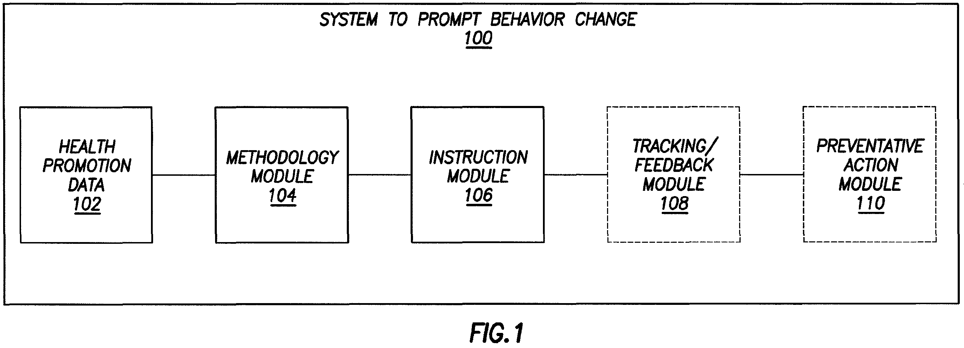

Referring now to **Figure 1****,** there is shown a schematic of a system to prompt behavior change 100, including **health promotion data** 102 generated and / or communicated from / to a device; **a methodology module** 104 associated with software / processor to identify at least one behavior change methodology associated with the health promotion data 102; and **an instruction module** 106 associated with a software / processor to initiate the identified at least one behavior change methodology. Optionally, and in various aspects, a **tracking** / **feedback module** 108 may track and / or provide feedback associated with the health promotion data 102, the behavior change methodology, the initiation of the behavior change methodology, etc. Optionally, and in various aspects, a **preventative action module** 110 may initiate a preventative or other action.

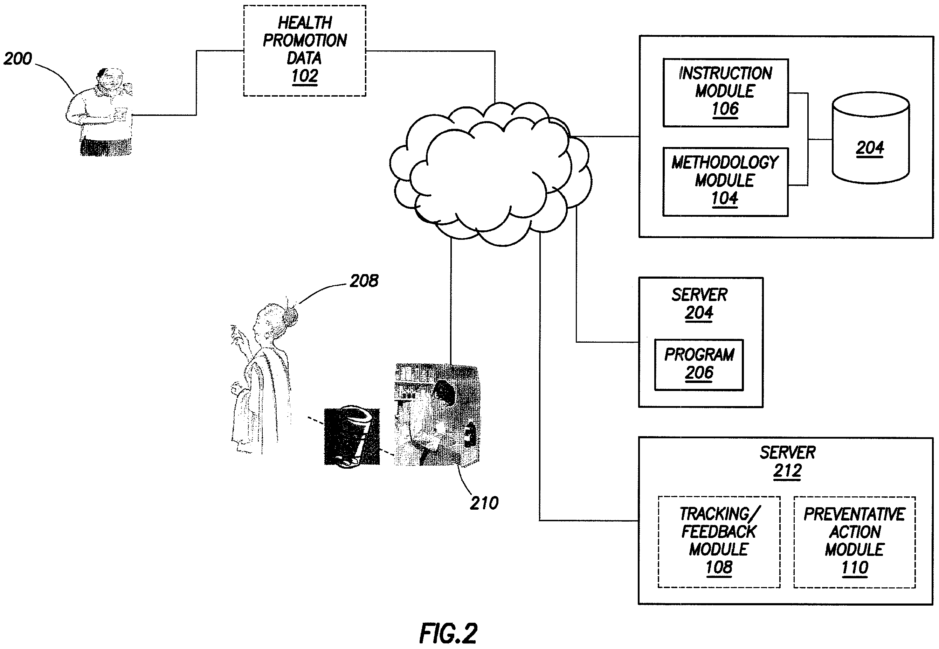

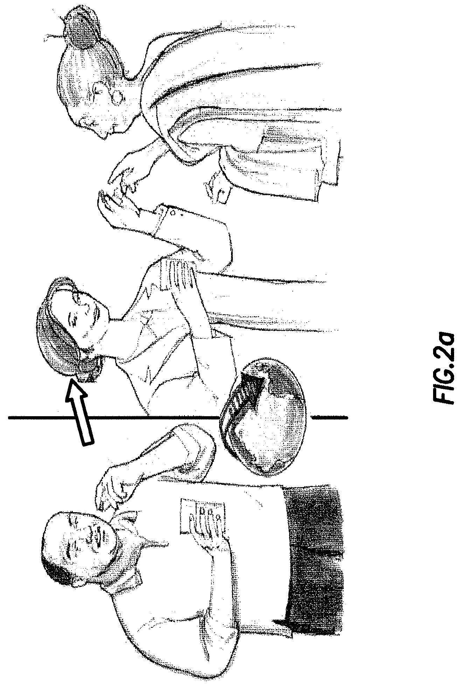

For example, and with reference to **Figures 2** **and** **2a****,** where there are shown a system operation of the system to prompt behavior change 100 of **Figure 1** and one conceptualization of a medmatch methodology, respectively. With continuing reference to the foregoing medmatch methodology illustration, the Boston patient 200 ingests a device-enabled medication (not shown), which communicates health promotion data 102 such as the time of ingestion and patient identification information to the patient's detector device (not shown). The detector device, for example, may be implemented as an on-body, adhesive communication patch (not shown). In this example, the detector device also collects health promotion data such as physiologic data, e.g., heart rate, heart variability, angle of repose, etc.

The detector device forwards the combined health promotion data 102 to the patient's mobile phone for onward communication to a hub, shown herein as first server 202, which includes the methodology module 104, instruction module 106, and a database 204.

Various aspects include the server 202, or other such hub device. As used herein, the term **hub** includes any hardware device, software, and / or communications component(s), as well as systems, subsystems, and combinations of the same which generally function to communicate the health promotion data 102. Communication of the health promotion data 102 includes receiving, storing, manipulating, displaying, processing, and / or transmitting the health promotion data 102. In various aspects, the hub also functions to communicate, e.g., receive and transmit, non-health promotion data. Broad categories of hubs include, for example, base stations, personal communication devices, and mobile telephones. Examples of a hub and other devices are discussed in U.S. Patent Application No. 12/522,249 filed July 02, 2009 entitled "INGESTIBLE EVENT MARKER DATA FRAMEWORK", *infra.*

The methodology module 104 processes the health promotion data 102 which include an identifier for the Boston patient 200. Of note, the health promotion data, the methodology module, and / or other system components may use personal identifiers such as name, etc., anonymous identifiers such as assigned numbers, or other identifiers to determine whether the health promotion data are associated with one or more methodologies. The methodology module 104 uses the identifier to compare with stored information in the database 204 to determine if the Boston patient 200 is a participant in one or more methodologies. In this example, the methodology module 104 processes the health promotion data 102 and identifies the data as associated with the Boston patient 202 and as a participant who has selected medmatch as his charitable giving program.

The instruction module 106 initiates, on a data system, shown herein as second server 204, a program 206 to facilitate donation of a medication to the Nairobi patient 208. The program 206 may be, for example, one or more software applications which provide one or more functions necessary to administer a methodology, e.g., a donation. For example, a medmatch software application may interact with one or more networks of systems, systems, system components, and / or devices to provide information regarding updates on donations to recipients, e.g., identity of recipient, type of medication, date and method of delivery to recipient, pharmacy 210 providing medication, dosing instructions for recipient, manufacturers / donors responsible for the donations, etc.

In various aspects, one or more hubs store, manipulate, and / or forward, directly or indirectly, the health promotion data 102, alone or in combination with other data, to one or more data systems. The data systems include any hardware device, software, and / or communications component, as well as systems and subsystems of the same, which generally function to provide a service or activity related to the health promotion data 102, e.g., program 206 which provides instructions for the medmatch software application.

The example further includes a third server 212 having the tracking / feedback module 108 and the preventative action module 110. Acting independently of or interoperatively with one another, the tracking / feedback module 108 and preventative action module 110 receive data from any one or more variety of sources, e.g., the patient's mobile phone, the methodology module 104, the instruction module 106, the program 206, the pharmacy system 210, and / or other sources.

The tracking/feedback module 108 receives, collects, etc., data regarding individual donors, groups of donors, causes donated to, etc., and provides feedback relevant to one or more methodologies to networks, computers, devices, etc., associated with various interested parties, e.g., the donor, the manufacturer, and the recipient.

The preventative action module 110 receives, monitors, processes, etc., data regarding boundary conditions, events, etc. relevant to use of the system. One such category of data is the monitoring for proper ingestion of prescribed medication to avoid over-ingestion, under-ingestion, improper dosage times, etc. For example, the health promotion data 102 may include information regarding the type and dosage frequency of a particular medication. The preventative action module 110 may compare such data against data stored in the database 204 having dosage instructions for the Boston patient 200 and for the Nairobi patient 208. Upon identification of a discrepancy, the preventative action module may generate appropriate alerts, communications, etc., to the patient, to the health care provider(s), to the pharmacy 210, etc. to ensure dosing is brought back into conformance with or remains within prescribed regimens.

The health promotion data include any and all data related to promoting, maintaining, establishing, improving, etc., the health of an individual. Health promotion data explicitly includes data that are machine-compatible, i.e., capable of being generated by, read by, written to, stored on or within, communicated from or to, and/or processed by a tangible machine or machine component, i.e., automatable data. Examples of machines and machine components include networks of computers, computers, storage media, communication devices, processing devices, circuitry, etc., as may be now known or provided in the future. Examples of data content include user identification; type, manufacturer, amount, time, and mode of delivery of products, e.g., medications, placebos, vitamins, foodstuff, etc. Examples of mode of delivery include ingestion, injection, inhalation, infusion, transdermal, insertion. Examples of devices that generate health promotion data include ingestible devices; intelligent syringes; intelligent IV bags; intelligent inhalers; intelligent infusers and catheters; data receivers and detectors, e.g., personal health companions;, smart packaging, memory and reminder tools; blood pressure cuffs; scales, glucometers, exercise tools and devices, eating habit trackers, medical and hospital devices, and other health-promoting devices. Examples of smart syringes and injection events, for example, include those discussed in U.S. Patent Application No. 12/673,326 filed February 12, 2010 entitled "BODY-ASSOCIATED RECEIVER AND METHOD", *infra.* Examples of intelligent inhalers and inhalation events include those discuss in U.S Provisional Patent Application No. 61/373,803 filed August 13, 2010 entitled "SYSTEM AND METHOD FOR DELIVERY AND DETECTION OF AN INHALABLE DOSE", *infra.* As used herein, the term "health-promoting devices" means any device, component, etc., capable of precise measurement of one or more health related parameters, e.g., heart rate, heart rate variability, angle of repose, accelerometer data, ingestion event, injection event, inhalation event, infusion event, drug depot release event, etc. This is in contrast to more subjectively-derived data such as patient-entered estimates of measurements, events as recorded by patients, etc.

Various aspects extend to non-medication and medication-like medical devices and monitoring products, where the adherence to a medical, health and wellbeing-related regimen can link a personal decision or choice to an altruistic goal and associated donation, show of support etc. For example, the wearing and / or use of a hearing aid, a pedometer, a weight scale, a blood pressure cuff, a blood glucose meter, etc., may all enable medmatch: any sensor-enabled measurement of personal decisions, choices and physiologic state, such as sensed parameters of heart rate, sleep, activity, respiration, diet and molecular parameters such as blood glucose, cholesterol, creatinine, etc., may be linked to medmatch. Another example is any assessment of food consumption and caloric intake enabling medmatch, where a target goal (such as lower caloric intake) drives the medmatch process. Another example is smoking cessation, where devices that demonstrate a decline in cigarettes or related products being consumed enable medmatch to make donations for every product *not* consumed by the individual.

Examples of the foregoing devices include, but are not limited to, those described in:
U.S. Patent Application No. 11/912,475 filed June 23, 2008 entitled "PHARMA - INFORMATICS SYSTEM", U.S. Patent Application No. 12/404,184 filed March 13, 2009 entitled, "PHARMA-INFORMATICS SYSTEM", U.S. Patent Application No. 12/522,249 filed July 02, 2009 entitled "INGESTIBLE EVENT MARKER DATA FRAMEWORK"U.S. Patent Application No. 12/741,583 filed on May 05, 2010 and entitled "HIGH-THROUGHPUT PRODUCTION OF INGESTIBLE EVENT MARKERS", PCT Patent Application No. PCT/US10/34186 filed on May 10, 2010 and entitled "INGESTIBLE EVENT MARKERS COMPRISING AN IDENTIFIER AND AN INGESTIBLE COMPONENT", U.S. Patent Application Serial No. 12/238,345 entitled, "IN-BODY DEVICE WITH VIRTUAL DIPOLE SIGNAL AMPLIFICATION" filed September 25, 2008, U.S. Patent Application No. 12/744,642 filed on April 27, 2010 and entitled "HIGHLY RELIABLE INGESTIBLE EVENT MARKERS AND METHODS OF USING SAME", U.S. Patent Application No. 12/238,345 filed September 25, 2008 and entitled "IN-BODY DEVICE WITH VIRTUAL DIPOLE SIGNAL AMPLIFICATION", U.S. Patent Application No. 12/564,017 filed September 21, 2009 entitled "COMMUNICATION SYSTEM WITH PARTIAL POWER SOURCE, U.S. Patent Application No. 12/673,326 filed February 12, 2010 entitled "BODY-ASSOCIATED RECEIVER AND METHOD", PCT application serial no. PCT/US2007/082563 entitled CONTROLLED ACTIVATION INGESTIBLE IDENTIFIER, PCT application serial No. PCT/US2007/024225 entitled ACTIVE SIGNAL PROCESSING PERSONAL HEALTH SIGNAL RECEIVERS, PCT application serial no. PCT/US2007/022257 entitled LOW VOLTAGE OSCILLATOR FOR MEDICAL DEVICES, PCT application serial no. PCT/US2008/052845 entitled INGESTIBLE EVENT MARKER SYSTEMS, PCT application serial no. PCT/US2008/053999 entitled IN-BODY POWER SOURCE HAVING HIGH SURFACE AREA ELECTRODE, PCT application serial no. PCT/US2008/056296 entitled IN-BODY POWER SOURCE HAVING MULTIDIRECTIONAL TRANSMITTER, PCT application serial no. PCT/US2008/056299 entitled IN-BODY POWER SOURCE HAVING DEPOLYABLE ANTENNA and PCT application serial no. PCT/US2008/077753 published as WO 2009/042812; the disclosures of which applications are herein incorporated by reference, U.S. Patent Application No. 11/912,475 filed April 28, 2006 entitled "PHARMA-INFORMATICS SYSTEM", U.S. Patent Application No. 12/522,249 filed July 02, 2009 entitled "INGESTIBLE EVENT MARKER DATA FRAMEWORK", U.S. Patent Application No. 12/349,453 filed Jan 06, 2009 entitled "SMART PARENTERAL ADMINISTRATION SYSTEM", U.S. Patent Application No. 12/776,480 filed July 11, 2007 entitled "ACOUSTIC PHARMA-INFORMATICS SYSTEM", and U.S Provisional Patent Application No. 61/373,803 filed August 13, 2010 entitled "SYSTEM AND METHOD FOR DELIVERY AND DETECTION OF AN INHALABLE DOSE". Each of the foregoing is incorporated by reference in its entirety.

The methodology module and the instruction module include any implementation of software, hardware, firmware or combinations of the foregoing, whether standalone, integrated with other modules or multiple devices, etc., so long as the modules are capable of carrying out the functions described herein. Either or both of the modules may be associated with, i.e., may be resident on, executable by, displayable by, etc., a component, a device, a computer, a network or networks of communicating devices, etc.

The behavior change methodologies include many and varied methodologies. In addition to the illustrated MEDMATCH methodology, examples include RACES WITHIN REACH methodology; PICK A DESKTOP WIDGET / AVATAR methodology; FAMILY RESPONSIBILITY methodology; VIRTUAL MANSION methodology; DAILY HATCH methodology; FITIMALS methodology; DELIGHTFUL COMPARATORS methodology; ADHERE TO WIN methodology; THE SHAME GAME methodology; PLEDGE MATCHING methodology; HELP FROM MY FRIENDS methodology; LOVE BUZZ methodology; PATCH ALERTS methodology; DONE! BUZZ methodology; PLUG methodology; REAL PATIENT PROFILES methodology; MOOD MINER methodology; HEART FIT methodology; THE SWIMMER PATCH methodology; SMALL STEPS TO BIG RESULTS methodology; COMMIT TO HEALTHY EATING methodology; and MATCHED methodology. The foregoing non-exhaustive list is provided as an illustration of some of the variety and versatility of the system, and not as a limitation thereof. Each of the methodologies is described in detail hereinafter.

### RACES WITHIN REACH Methodology

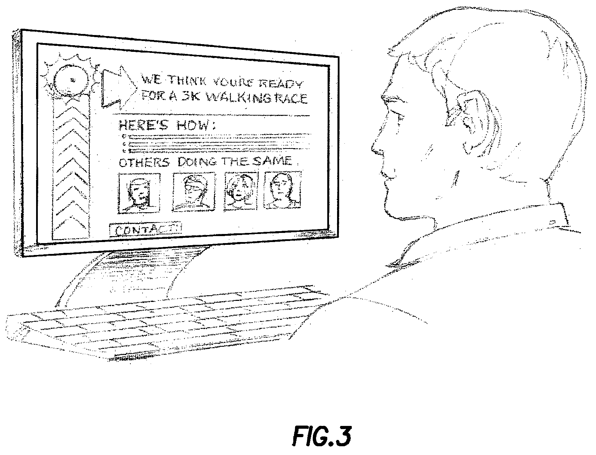

The races within reach methodology, as conceptually illustrated in **Figure 3****,** may prompt behavior change by encouraging the user with suggestions of participation in various organized fitness events. As the user's fitness improves, the system suggests fitness events appropriate for their abilities and provides training guidance as the event approaches. Additionally, other people training for the same event at a similar performance level can be connected for group training or support. Placing new fitness activities in the path of users creates fitness nudges that are further strengthened by connecting groups of participants matched according to capabilities.

Examples of implementation include a participant providing information about his abilities (health promotion data) to a website associated with the methodology module. The methodology module may process the health promotion data to identify the corresponding races within reach methodology. The instruction module may initiate an instruction whereby program(s) and associated system(s) analyze the participant's abilities, select fitness events and the like based on the analyzed abilities, and make the event information accessible by the user, e.g., display results, updates, etc., on a display device.

### PICK A DESKTOP WIDGET / AVATAR Methodology

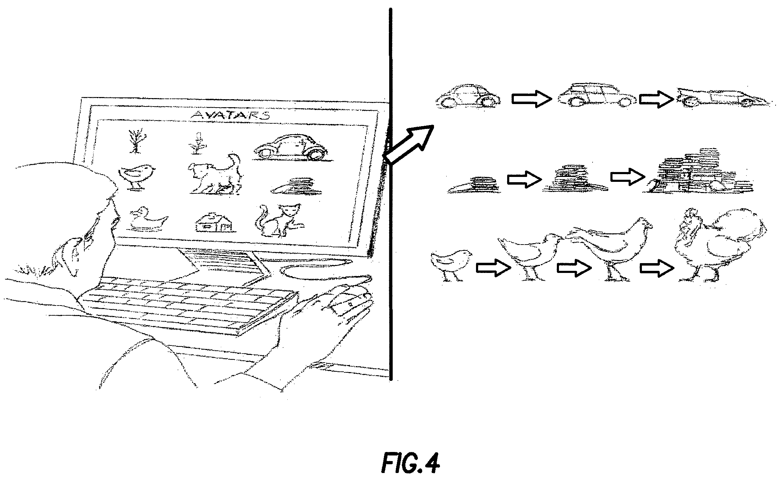

The pick a desktop widget / avatar methodology, as conceptually illustrated in **Figure 4****,** may provide a virtual representation of the status of each member of a family, based on their adherence to an exercise program, medication regimen, sleep schedule, or composite and displayed on their smart phone or computer desktop, etc. The user chooses which avatar they wish to represent their status and as it changes (with exercise, adherence, etc.), the avatar morphs to visually reflect this change. The avatars may create a visible mental model for states and changes that are invisible. The ability to select an avatar that is meaningful to the user creates relevance.

Examples of implementation include participants selecting avatars displayed by a software application. The selected avatars and corresponding participant information regarding exercise, adherence, etc. (health promotion data) are communicated to a website associated with the methodology module. The methodology module may process the health promotion data to identify corresponding pick a desktop widget / avatar methodology. The instruction module may initiate an instruction whereby program(s) and associated system(s) analyzes status changes and updates avatars to morph accordingly.

### FAMILY RESPONSIBILITY Methodology

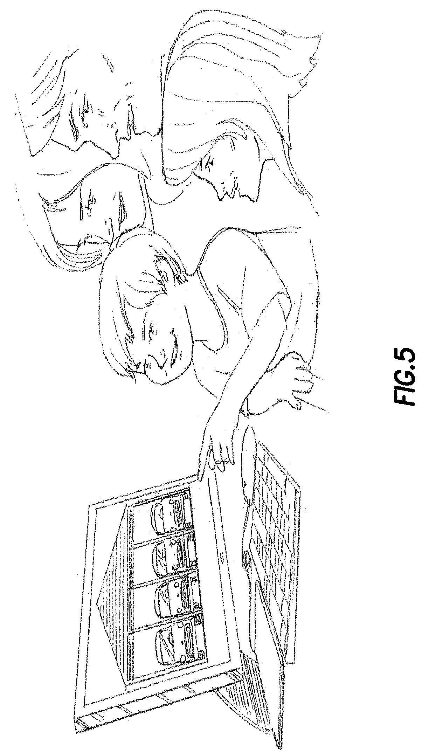

The family responsibility methodology, as conceptually illustrated in **Figure 5****,** provides a virtual representation of the status of each member of a family, based on their adherence to an exercise program, medication regimen, sleep schedule, composite, etc., and displayed, for example, on their smart phone or computer desktop. Each member of the family, for example, is represented by their own avatar, all linked by a common theme (such as multiple cars in a garage). The avatars visually morph to reflect changes in exercise, adherence, etc. and everyone participating can see the avatars of the others. The visibility of each other's avatars provides strong social reinforcers that motivate adherence. Game-like progression maintains interest, which becomes yet another reason for adherence and establishes new behavioral norms.

Examples of implementation include selection of avatars, e.g., each reflective of a common them, as displayed by a software application and generation of medication regimen data (health promotion data). The selected avatars and corresponding health promotion data are communicated to a website associated with the methodology module. The methodology module may process the health promotion data to identify the corresponding family responsibility methodology. The instruction module may initiate an instruction whereby program(s) and associated system(s) generate game applications, etc. displaying representations of relative adherence, progression, etc. by visual changes to the selected avatars.

### VIRTUAL MANSION Methodology

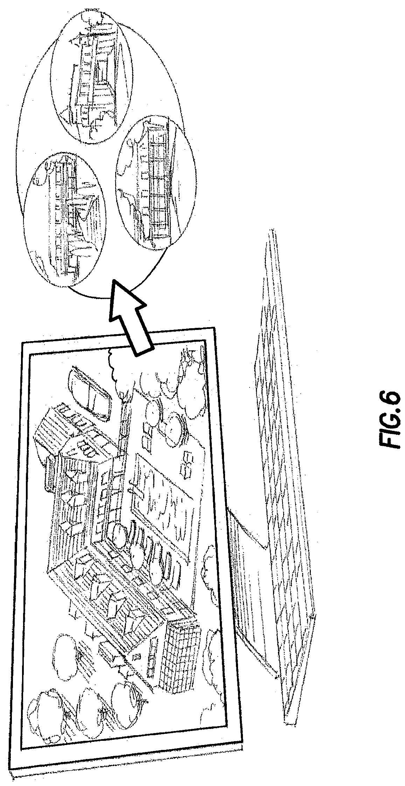

The virtual mansion methodology, as conceptually illustrated in **Figure 6****,** is an online, virtual representation of a group of people where each one is represented by an object in an online virtual mansion. As each person's status changes, e.g., through exercise, adherence, etc., their selected objects morph to reflect the real-life change by becoming more or less opulent, clean, etc. By helping to support other members of the mansion, the mansion itself may gain new features or compete with other mansions in the virtual neighborhood. Rolling several avatars into one community increases members' sense of responsibility to "do their part" to maintain and improve community health. Competition among communities further magnifies adherence.

Examples of implementation include generation of health promotion data. The health promotion data are communicated to a website associated with the methodology module. The methodology module may process the health promotion data to identify the corresponding virtual mansion methodology. The instruction module may initiate an instruction whereby program(s) and associated system(s) generate software options for the persons to select mansion attributes and other indicia pertinent to the virtual mansion. The software program may further correlate health promotion data updates with predetermined relative changes to the mansion attributes, and display such changes.

### DAILY HATCH Methodology

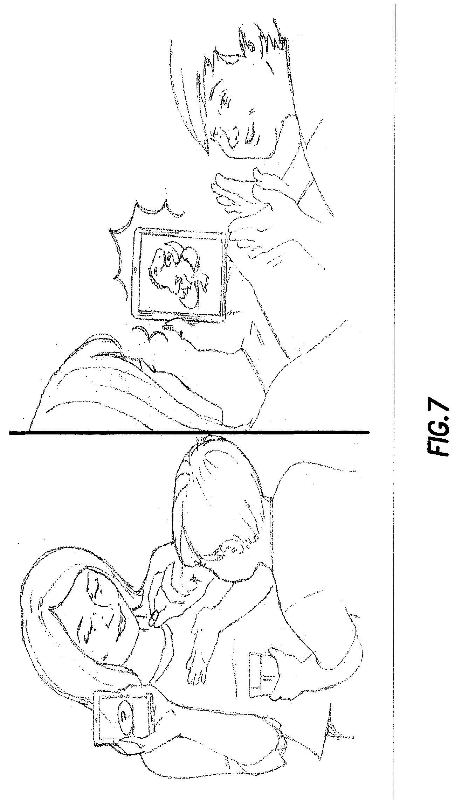

The daily hatch methodology, as conceptually illustrated in **Figure 7****,** provides an application, e.g., a computer or smart-phone application, which generates an enticement for children (or others) to engage with their medication. The application shows a delightful result when a child takes their medication. The result, for example, may display as a dinosaur hatching from an egg, a seashell opening up, etc. The anticipation about which animal will emerge shifts focus from an unpleasant dosing moment to delightful play.

Examples of implementation include generation of a medication delivery event, i.e., health promotion data. The health promotion data are communicated to a website associated with the methodology module. The methodology module may process the health promotion data to identify the corresponding daily hatch methodology. The instruction module may initiate an instruction whereby program(s) and associated system(s) identify predetermined health promotion events and generate software outputs relative to the event, e.g., display of a dinosaur hatching.

### FITIMALS Methodology

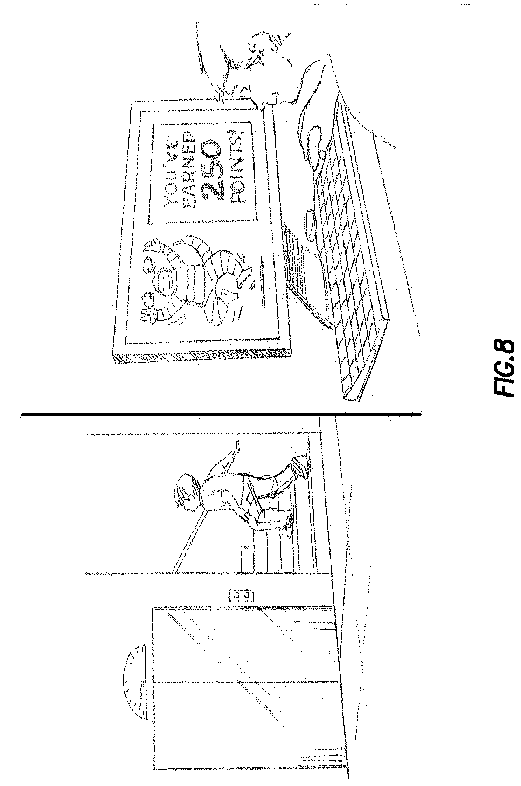

The fitimals methodology, as conceptually illustrated in **Figure 8****,** provides a virtual game world that reflects and responds to real-world logged events. A real world action such as fitness, medication adherence, etc, builds a virtual character's abilities and progresses them through a narrative. Tying a characters abilities and powers in an engaging virtual world to real life actions provides new incentives to make better choices in the real world.

Similarly, methodologies may include a "gamefit" model. The gamefit methodology may include, for example, games that attract and entertain a user. In some aspects, the game keeps rewarding the user; shows the user's progress; permits the user to become deeply involved with the game. To get the user through challenging times, for example, the gamefit methodology keeps challenging the user to continue and provides new ways for the user to interact with others.

Examples of implementation include generation of health promotion data. The health promotion data are communicated to a website associated with the methodology module. The methodology module may process the health promotion data to identify the corresponding fitimals methodology. The instruction module may initiate an instruction whereby program(s) and associated system(s) identify predetermined health promotion events, generates and builds a virtual character's abilities, and displays the character in conjunction with a predetermined narrative.

### DELIGHTFUL COMPARATORS Methodology

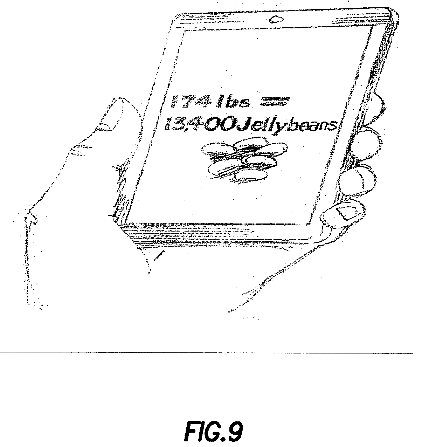

The delightful comparators methodology, as conceptually illustrated in **Figure 9****,** provides a scale that measures body weight without using pounds and kilograms. Weight is displayed in unique and delightful units such as jellybeans, billiard balls, blueberries, etc. Trying to lose weight may be a frustrating and slow process full of guilt and fear of failure. The dread of the weighing moment is lightened by including humorous and surprising units for weight. Additionally, prompting with smaller units (such as jellybeans) allows for more visible changes in weight.

Examples of implementation include generation of health promotion data, e.g., an individual's weight. The health promotion data are communicated to a website associated with the methodology module. The methodology module may process the health promotion data to identify the corresponding delightful comparators methodology. The instruction module may initiate an instruction whereby program(s) and associated system(s) identify predetermined health promotion events such as weight changes; generates the corresponding change in the humorous and surprising units; and displays the traditional units, the humorous and surprising units, and visual indicia of the surprising units.

### ADHERE TO WIN Methodology

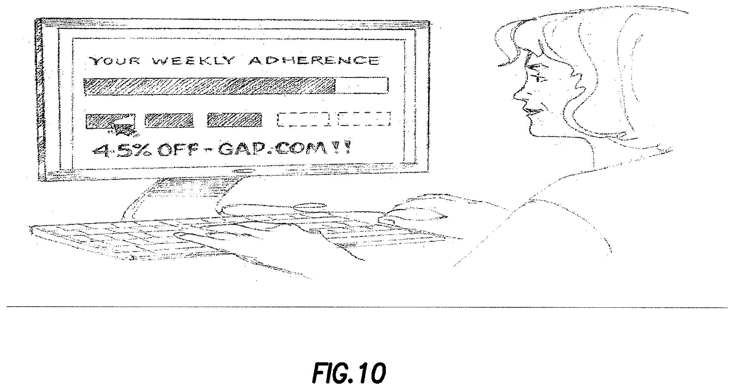

The adhere to win methodology, as conceptually illustrated in **Figure 10****,** provides desirable incentives for continued adherence in the form of prizes that users can win. High levels of adherence (to medication, exercise regimen, etc.) are rewarded with an increased number of chances to win discounts and prizes from personally relevant categories, for oneself or one's family. The perceived chance of winning motivates adherence. Additionally, prizes desired by family members add social pressure to further adhere.

Examples of implementation include generation of health promotion data, e.g., medication event data, exercise data, etc. The health promotion data are communicated to a website associated with the methodology module. The methodology module may process the health promotion data to identify the corresponding adhere to win methodology. The instruction module may initiate an instruction whereby program(s) and associated system(s) identify predetermined health promotion events qualifying for prizes and facilitate award of the prizes, e.g., by providing award details, total award information, update of other family awards, etc.

### THE SHAME GAME Methodology

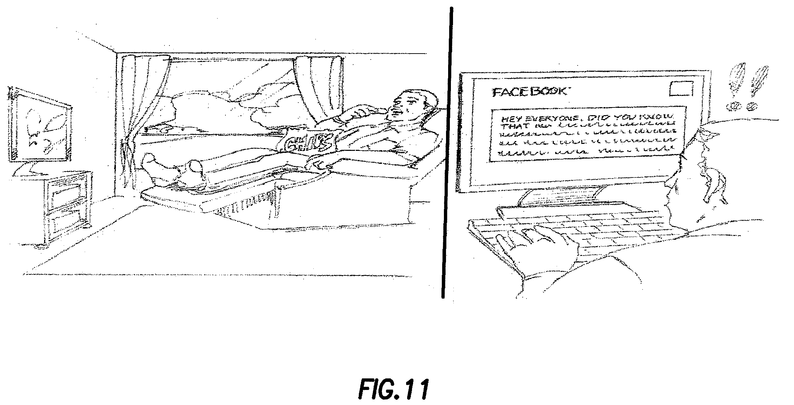

The shame game methodology, as conceptually illustrated in **Figure 11****,** provides an online program which uses social pressure to help a group of friends or family stick to their fitness or medication adherence goals. On sign up, all participants enter embarrassing information into the system about other members. Participants are enticed to stick to their goals with the knowledge that a lapse (after a warning, for example) will result in one of the embarrassing facts about them being posted to a social website, e.g., Facebook. Desire to avoid public shame may be a powerful motivator.

Examples of implementation include generation of health promotion data, e.g., medication event data, exercise data, etc. The health promotion data are communicated to a website associated with the methodology module. The methodology module may process the health promotion data to identify the corresponding shame game methodology. The instruction module may initiate an instruction whereby program(s) and associated system(s) identify predetermined health promotion events indicating nonconformance, generate a warning by text, email, etc. and, upon a second nonconformance event, post the previously store embarrassing-fact data to Facebook.

### PLEDGE MATCHING Methodology

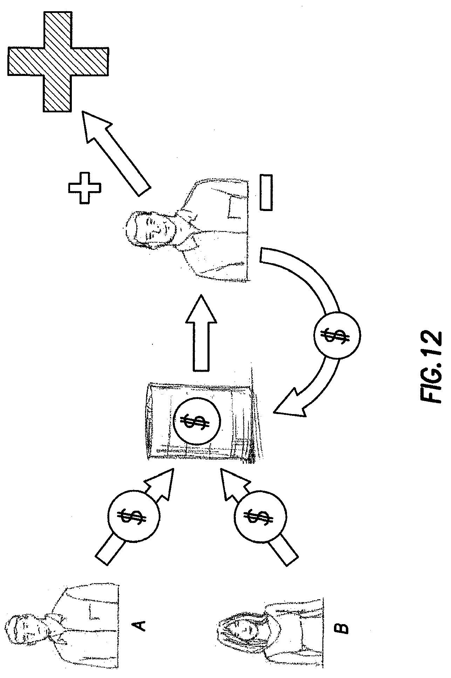

The pledge matching methodology, as conceptually illustrated in **Figure 12****,** provides a support program that uses pledge(d) money from friends or family as an enticement for an individual to stick to their fitness or medication adherence goals. A charity is chosen by the main participant and pledge contributions are made through the system. After the goal period has elapsed, the actual contribution to the selected charity may be calculated and provided via various means, e.g., the pledge may range from $0 (in the case of total non-adherence) to twice the pledged amount (in the case of total adherence). The matching funds may come from the general pledge pool, insurance company contributions, etc. Pledges create a public commitment to follow-through. Both the possibility of doubling one's donation and the prospect of "falling short" of one's goals further reinforces adherence.

Examples of implementation include generation of health promotion data, e.g., medication event data, exercise data, etc. The health promotion data are communicated to a website associated with the methodology module. The methodology module may process the health promotion data to identify the corresponding pledge matching methodology. The instruction module may initiate an instruction whereby program(s) and associated system(s) identify predetermined health promotion events indicating adherence and nonconformance events, calculate a total donations based on predetermined rules and the actual health promotion data, update adherence and pledge outcomes on various systems, and link in / communicated with preexisting pledge program systems to provide pledge data to such systems.

### HELP FROM MY FRIENDS Methodology

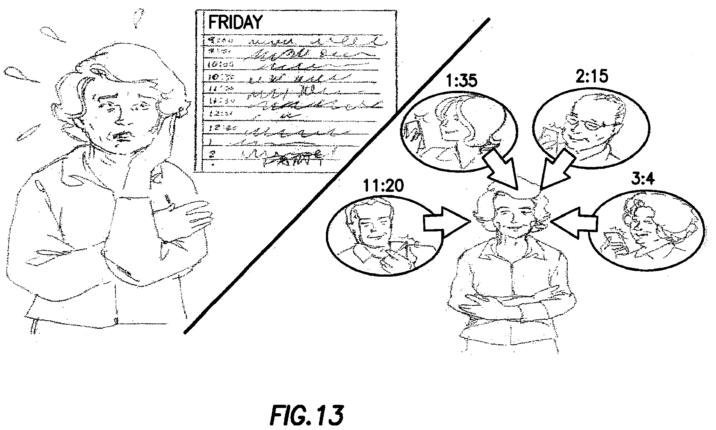

The help from my friends methodology, as conceptually illustrated in **Figure 13****,** provides a system that facilitates the engagement of support from an individual's personal network by, for example, taking medication with an ingestible device that marks the ingestion event. If the individual feels that they need (or might need) support, the user swallows a pill that alerts their designated network to this fact through texts, Facebook alerts, etc. Their friends/family are then able to provide help, encouragements, or empathy. The automatic and effortless activation of a support network by ingesting an event marker helps individuals navigate through adherence rough spots and potentially prevents slipping.

Examples of implementation include generation of health promotion data, e.g., medication event data. The health promotion data are communicated to a website associated with the methodology module. The methodology module may process the health promotion data to identify the corresponding help from my friends methodology. The instruction module may initiate an instruction whereby program(s) and associated system(s) generate meaningful alerts, messages, etc.

### LOVE BUZZ Methodology

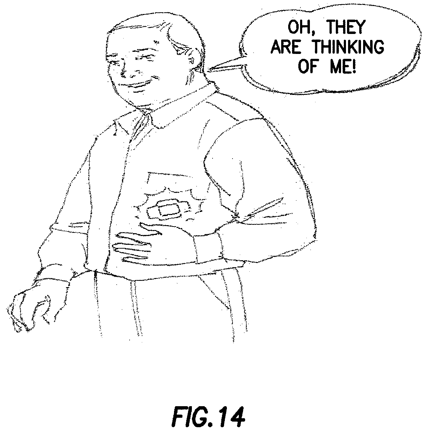

The love buzz methodology, as conceptually illustrated in **Figure 14****,** provides a vibration-enabled detector such as an adhesive communication device that privately signals that another is thinking about you. A user's loved ones are able to send a buzz as a signal that they are thinking of the user. Through the use of vibration patterns, the buzz may indicate different senders and multiple messages, e.g. Morse code. A buzz is like a remote hug from loved ones, sending a caring signal at unexpected moments that creates an emotional connection to the patch.

Examples of implementation include generation of health promotion data, e.g., loved one's data. The health promotion data are communicated to a website associated with the methodology module. The methodology module may process the health promotion data to identify the corresponding love buzz methodology. The instruction module may initiate an instruction whereby program(s) and associated system(s) generate vibratory or other messages, e.g., blinking lights, on the recipient's detector. Examples of a detector include those discussed in U.S. Patent Application No. 12/673,326 filed February 12, 2010 entitled "BODY-ASSOCIATED RECEIVER AND METHOD, *supra.*

### PATCH ALERTS Methodology

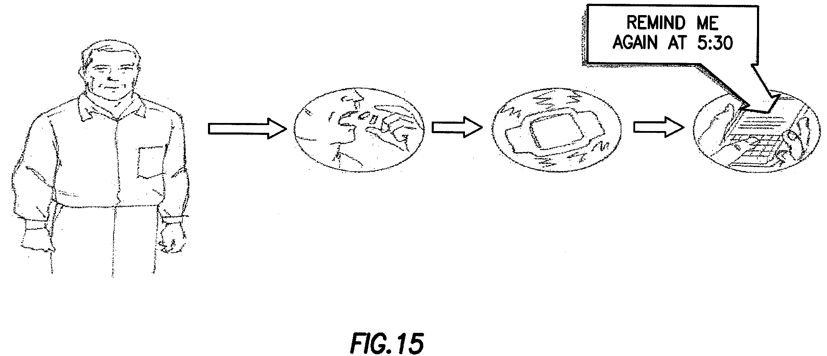

The patch alerts methodology, as conceptually illustrated in **Figure 15****,** utilizes a vibration-enabled detector, e.g., adhesive patch, to privately alert the user that she forgot to take her medication. The patch vibrates to let the user know that she forgot to take their medication. Should she be away from the medication, she can reset a reminder function. Reminders from the patch create trust that the patch is working in addition to aiding adherence and from the user's perspective gives the patch a meaningful role, alone or in addition to other detector functionality.

Similarly, a "patch communicator" methodology may coach a user in real time and enhance the value of the patch. Other inclusions in this methodology include, for example, generating alerts based on short-term successes to reinforce the user's actions and help the user to remember. The patch communications, e.g., audio, visual, tactile, etc., may constantly reinforces healthy choices and enable the user to track their behavior.

Examples of implementation include generation of health promotion data, e.g., data generated by a software program associated with ingestion event data that identifies failure to receive data indicating on on-schedule ingestion event. The health promotion data are communicated to a website associated with the methodology module. The methodology module may process the health promotion data to identify the corresponding patch alerts methodology. The instruction module may initiate an instruction whereby program(s) and associated system(s) generate vibratory or other messages, e.g., blinking lights, on the recipient's detector, indicating that the user may want to determine if a dose has been missed.

### DONE! BUZZ Methodology

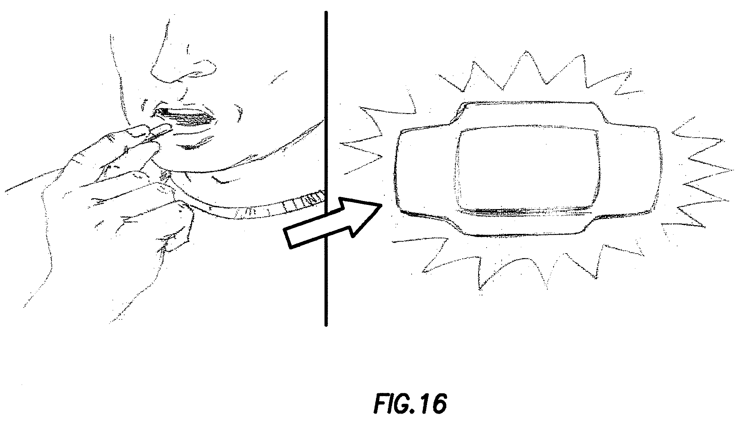

The done! buzz methodology, as conceptually illustrated in **Figure 16****,** provides a vibration-enabled detector, e.g., adhesive patch, to privately confirm to the user that the medication was swallowed. Upon ingestion of a medication, the system provides the user with feedback that the medication has been registered, building trust in the technology. Receiving a buzz when the pill is registered may satisfy the user's craving for certainty that the system is working and gives the patch a(nother) meaningful role in the system.

Examples of implementation include generation of health promotion data, e.g., ingestion event data. The health promotion data are communicated to a processor associated with the methodology module. The methodology module may process the health promotion data to identify the corresponding done! Buzz methodology. The instruction module may initiate an instruction whereby program(s) and associated system(s) generate vibratory or other messages, e.g., blinking lights, on the recipient's detector.

### PLUG Methodology

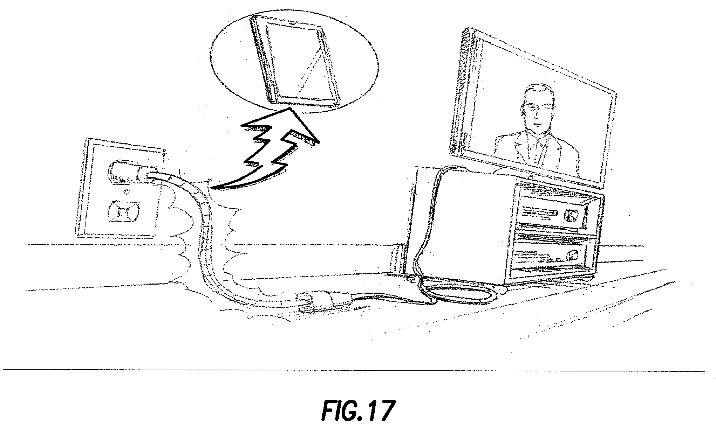

The plug methodology, as conceptually illustrated in **Figure 17****,** provides a smart outlet that enables the system to toggle power to and monitor usage of various small appliances. The user chooses an appliance (such as a coffee maker, TV, or video game system) to connect to the smart outlet and selects usage thresholds, e.g., 100% adherence yields ten weekly hours of TV. Levels of adherence, exercise, etc. logged by the system determines how much time the user can spend with the selected appliance. In various aspects, the smart outlet may also function as a base station or hub. Self-imposed "handcuffs" that prevent access to an electronic device provide a powerful incentive to adhere. This may turn long-term, decoupled effects of not adhering into current consequences.

Examples of implementation include generation of health promotion data, e.g., ingest event data. The health promotion data are communicated to a hub associated with the methodology module. The methodology module may process the health promotion data to identify the corresponding plug methodology. The instruction module may initiate an instruction whereby program(s) and associated system(s) monitor and control access to the selected appliances according to the preselected conditions.

### REAL PATIENT PROFILES Methodology

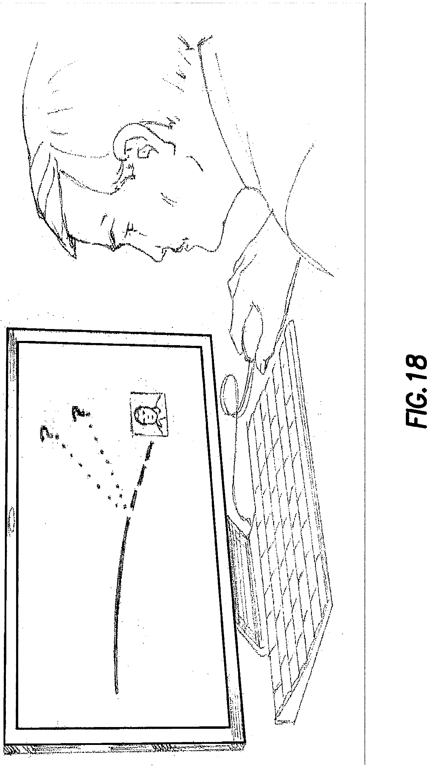

The real patient profiles methodology, as conceptually illustrated in **Figure 18****,** provides an element of feedback to an individual's treatment / regimen for conditions that might otherwise have long feedback loops or none at all. Current actions are forecasted based on physiology and adherence, showing the user a real (and perhaps curated) profile of somebody who once was where the user currently is. For predictions that might be discouraging, the system provides suggestions on behavior and alternative inspirational profiles. Predicting the individual's future health states using real patient profiles creates a visceral connection between today's actions and tomorrow's outcomes and, therefore, more persuasive reasons to change one's actions.

Similarly, a "real futures" methodology may give a vicarious view of a user's potential future. For example, the methodology may entice a user to take control of their future by prompting them with tips from similarly-situated person. In this manner, the user is permitted to receive support and encouragement through objective data and is shown a possible change of trajectory to fuel the user's confidence.

Examples of implementation include generation of health promotion data, e.g., disease condition, level of treatment, duration of treatment, etc. The health promotion data are communicated to a website associated with the methodology module. The methodology module may process the health promotion data to identify the corresponding real patient profiles methodology. The instruction module may initiate an instruction whereby program(s) and associated system(s) generate suggestions, alternative inspirational profiles, etc.

### MOOD MINER Methodology

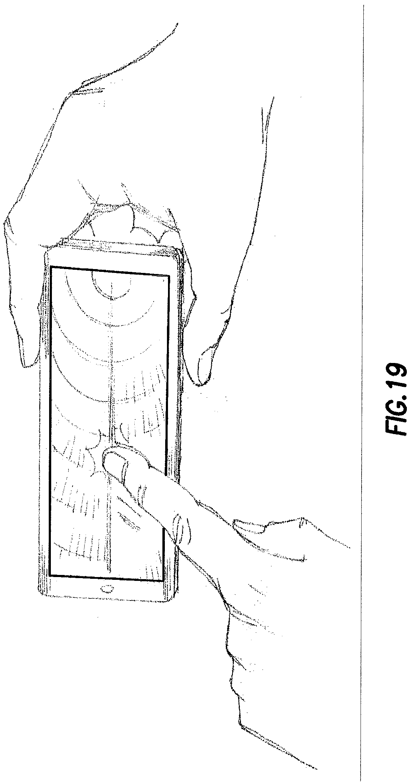

The mood miner methodology, as illustrated in **Figure 19****,** provides a system that collects objective measures as well as subjective inputs in a convenient way. Mood miner methodology identifies patterns and relationships between one's exercise, sleep, and medicine adherence with subjective inputs, making visible the relationships between them and making behavioral choices more relevant. Comparing longitudinal subjective input with objective body measures reveals surprising patterns that help correct intuitive theories about why the user feels the way he does. Adjusting his theories allows for stronger intrinsic motivators for better behavior.

Examples of implementation include generation of health promotion data, e.g., sleep data, medication ingestion data, and exercise data and subjective data. Subjective data generation may be accomplished via various methods. In one example, an application on the user's mobile phone displays concentric rings of varying colors. Each color is representative of the user's relative feelings, emotions, self-assessment, etc. The user selects the color(s) pertinent to the conditions and the application generates the subjective data. The health promotion data are communicated to the user's phone application, which has an associated methodology module. The methodology module may process the data to identify the corresponding mood miner methodology. The instruction module may initiate an instruction whereby program(s) and associated system(s) analyze the data for patterns and meaningfully display the patterns, related insights, etc.

### HEART FIT Methodology

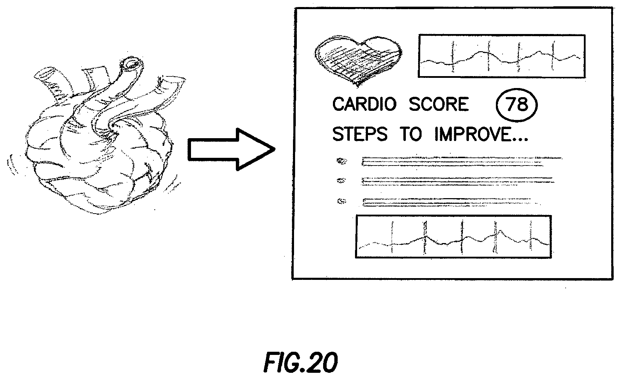

The heart fit methodology, as conceptually illustrated in **Figure 20****,** provides a system that focuses on heart metrics rather than analogs such as step counts for fitness or health goals. Using data such as resting heart rate and time spent above target heart rate, this system can provide information and guidance on fitness and daily exercise that span across activity. Also, the tracking of validated cardiovascular fitness metrics such as heart rate variability and heart rate time to recovery might provide insights for cardiology care. Many popular fitness tracking systems cannot measure reliable, long-term heart rate, which is arguably one of the most important measures of fitness. Moreover, increased insight into heart rate allows for richer fitness encouragement through a new set of metrics, i.e., other than traditional proxies for fitness such as weight, BMI, or exercise.

Examples of implementation include generation of health promotion data, e.g., heart rate, heart rate variability, fitness activity and resting data, etc. The health promotion data are communicated to a server associated with the methodology module. The methodology module may process the health promotion data to identify the corresponding heart fit methodology. The instruction module may initiate an instruction whereby program(s) and associated system(s) integrate data streams, fuse data to generate a refined cardiac (or other) health models, and infer treatment / regimen optimization steps, etc.

### SWIMMER PATCH Methodology

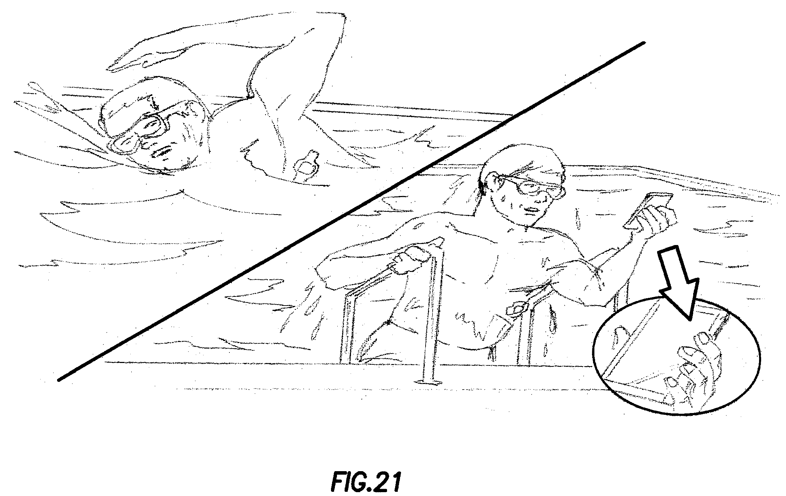

The swimmer patch methodology, as conceptually illustrated in **Figure 21****,** provides a system that focuses on heart metrics rather than analogs such as step counts for fitness or health goals. By monitoring heart rate in real time, the patch vibrates to alert the swimmer that they have reached their target heart rate, allowing optimal training or exercise. Additionally, by using accelerometer data, the system becomes a lap counter. This may also offer novel real-time tracking capabilities to swimmers across all ability levels. In terms of prompting behavior change, it is noted that this term is used broadly and includes concept(s) such as facilitating training and goal achievement. For example, the prompt to change behavior in using the swimmer patch may include delivery of data that prompt the swimmer to adhere to training regimens more closely, resulting in achievement of various swimming and training goals. A skilled artisan will recognize that multiple concepts apply.

Examples of implementation include generation of health promotion data, e.g., heart rate, accelerometer data, etc. The health promotion data are communicated to the patch having an associated with the methodology module. The methodology module may process the health promotion data to identify the corresponding swimmer patch methodology. The instruction module may initiate an instruction whereby program(s) associated with the patch generate vibratory or other messages, e.g., blinking lights, data display, etc., on the recipient's detector.

### SMALL STEPS TO BIG RESULTS Methodology

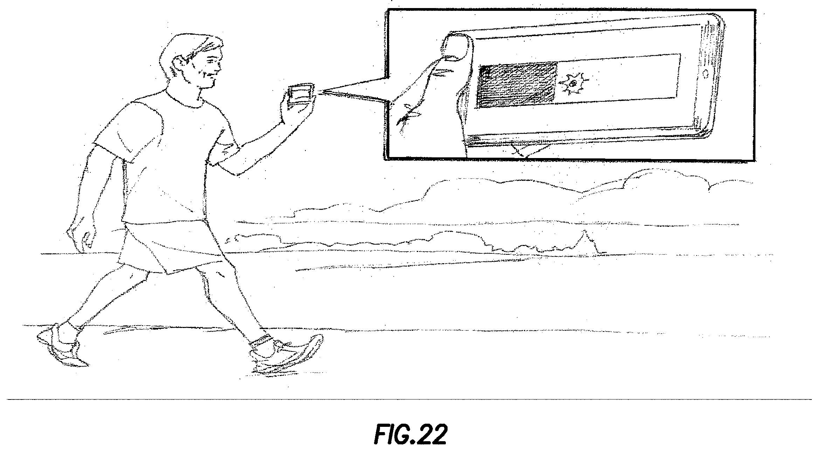

The small steps to big results methodology, as conceptually illustrated in **Figure 22****,** encourages physical activity by setting the goal of merely surpassing one's previous scores. By tracking information on physical activity, the system visually shows users their prior performance and guides them to beat it by exercising to move the representational dot past the threshold line. Any user who lapses is guided back through manageable and encouragingly gradual goal-setting. Transforms imposing long- term goals into smaller, more achievable goals.

Examples of implementation include generation of health promotion data, e.g., physical activity data, etc. The health promotion data are communicated to a hub, e.g., the user's mobile phone, associated with the methodology module. The methodology module may process the health promotion data to identify the corresponding small steps to big results methodology. The instruction module may initiate an instruction whereby program(s) associated with the system retrieve stored data of prior performances, compare it to the current health promotion data, and display the results via encouraging visual displays, etc.

### COMMIT TO HEALTHY EATING Methodology

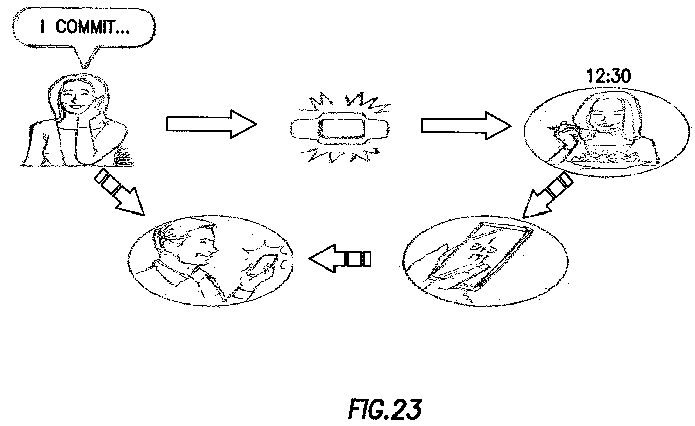

The commit to healthy eating methodology, as illustrated in **Figure 23****,** provides a system that encourages healthy eating habits through commitment pills and reminders. At the start of the day, the user takes pills, e.g., placebos, vitamins, etc., having associated ingestible devices, that represent their commitment to eating a certain number of vegetable or fruit servings and this commitment is noted on social media, e.g., her Facebook page. As each mealtime arrives, her patch vibrates to remind her of her commitment and upon acknowledging that she has eaten properly, the achievement is posted (celebrated) on her Facebook wall. By presenting commitments publicly, users feel more obligated to complete them. Additionally, planning healthy eating behaviors at the start of each day enables users to become more mindful about diet.

Similarly, a "placebo pills" methodology provides a system of "intention pills" e.g., placebos, vitamins, etc. that serve as an indicator or reminder of a user's intent, commitment, etc. The intention pill may be ingested as an individual program, as part of group participation, an empathy relay, a treatment simulation pill, etc. To illustrate, a user may commit to ingesting, and ingest, a placebo each time a friend has to ingest a prescribed medication for a treatment regimen. The commitment to "co-ingest" and the act of solidarity both shows a real support of the friend's plight and progress as well as helps the user offer continual support to the friend.

The intention pill may trigger thing to change for the user, make intentions of the user tangible to them and others, make the user feel empowered, enable the user to reach their goal, etc. Group participation may permit the user to feel as if they are part of a bigger cause, e.g., group invitation, reinforce the user's reason(s) for participating, and help the user connect to like-minded people. The empathy relay may provide support for a user's friend, give the user something to do for a friend, show the user's support, offer continual support for the friend, etc. The treatment simulation pill may assist the user in avoiding behavior, choices, etc., that worsen the user's condition, assist the user in experiencing the ramification of a treatment regimen in their life, give the user a reason to change their behavior to avoid potential consequences associated with foregoing a behavior change, etc. The placebo pills methodology may enhance behavior changes, etc., for persons

Examples of implementation include generation of health promotion data, e.g., ingestion event data. The health promotion data are communicated to a website associated with the methodology module. The methodology module may process the health promotion data to identify the corresponding commit to healthy eating methodology. The instruction module may initiate an instruction whereby program(s) and associated system(s) generate a comment on Facebook relevant to the commitment, generates timely and meaningful reminders, and, upon predetermined conditions, post celebratory comments on Facebook regarding the accomplishment.

### MATCHED Methodology

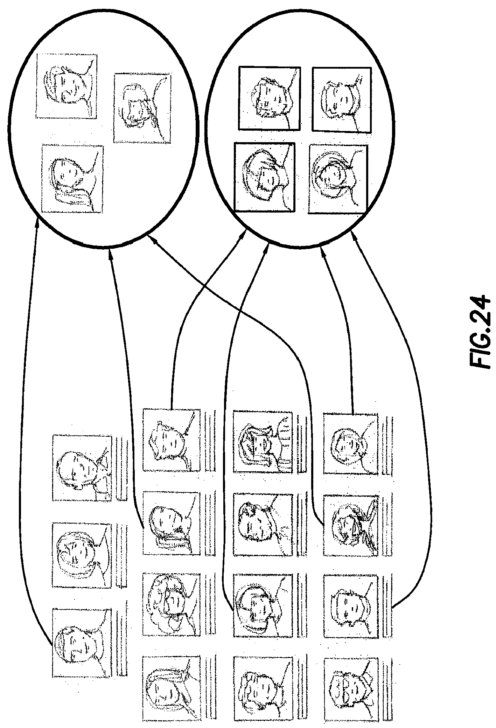

The matched methodology, as conceptually illustrated in **Figure 24****,** provides a system that selects people to form optimal support groups based on ailments, goals, needs, etc. Using information on user's adherence to medication and fitness regimens as well as collected personality data, this system groups people with similar (or potentially complementary) support needs to form inspirational and relatable groups for maximum benefit. Offering users a meaningfully matched support group both increases the potential for the group to be effective, and possibly alleviates fears of joining a group with an unknown composition.

Examples of implementation include generation of health promotion data, e.g., user adherence data, etc. The health promotion data are communicated to a website associated with the methodology module. The methodology module may process the health promotion data to identify the corresponding matched methodology. The instruction module may initiate an instruction whereby program(s) and associated system(s) match the user to group(s) of persons based on predetermined criteria, e.g., disease condition, fitness regimen, medication regimen, etc. and facilitate an online networking forum between support group members.

### Method

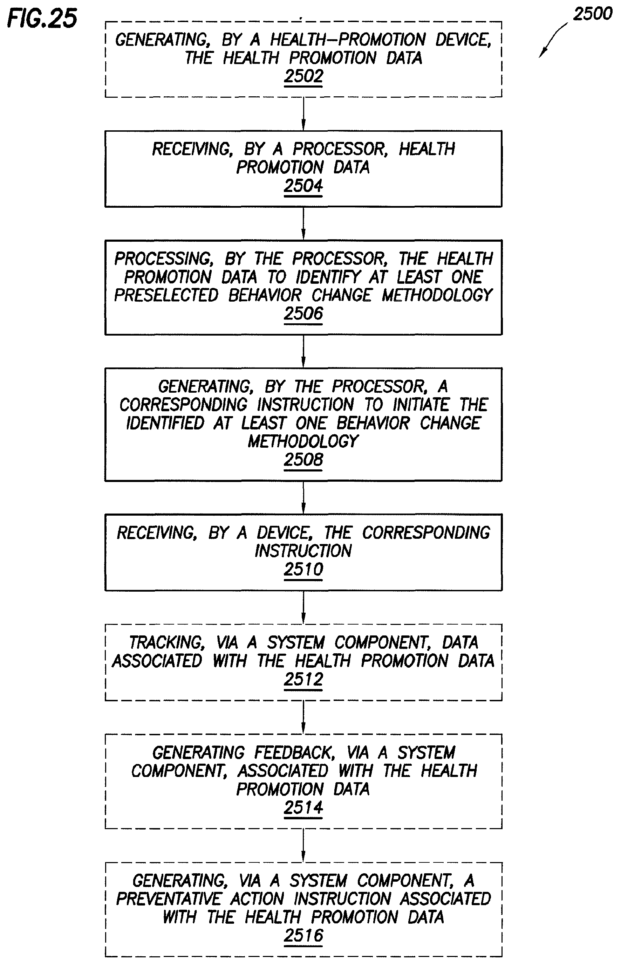

With reference now to **Figure 25****,** there is shown a method to prompt behavior change 2500. In various aspects, the method may optionally (illustrated in phantom outline) consist of an initial step of generating, by a health-promotion device, the health promotion data at step 2502. In various aspects, the method may consist / comprise steps of: receiving, by a processor, health promotion data at step 2504; processing, by the processor, the health promotion data to identify at least one preselected behavior change methodology at step 2506; and generating, by the processor, a corresponding instruction to initiate the identified at least one behavior change methodology at step 2508. In various aspects, the method may optionally consist or comprise of one or more of the following steps: receiving, by a device, the corresponding instruction at step 2510; tracking, via a system component, data associated with the health promotion data at step 2512; generating feedback, via a system component, associated with the health promotion data at step 2514; and generating, via a system component, a preventative action instruction associated with the health promotion data at step 2516.

In various aspects, and as heretofore discussed, the health promotion data may be associated with various health-related events and combinations thereof, e.g., an ingestion event, an injection event, an inhalation event, an infusion event, a health monitoring event, an physical activity event, and an eating event. To illustrate, the health promotion data related to an ingestion event may be generated by an ingestible device such as an RFID-enabled device, a current-altering device, etc.

The behavior change methologies include various examples of methodologies which function to prompt behavior, e.g., a desirable, sustainable behavior change associated with a health-related issue, event, regimen, etc.; to engender empathy, e.g., identify with a cause, garner family support, etc., which may prompt a behavior change, etc. The non-exhaustive list of examples previously discussed may be applied in various aspects of the method, i.e., a medmatch methodology; a races within reach methodology; a pick a desktop widget / avatar methodology; a family responsibility methodology; a virtual mansion methodology; a daily hatch methodology; a fitimals methodology; a delightful comparators methodology; an adhere to win methodology; a shame game methodology; a pledge matching methodology; a help from my friends methodology; a love buzz methodology; a patch alerts methodology; a done! buzz methodology; a plug methodology; a real patient profiles methodology; a mood miner methodology; a heart fit methodology; a swimmer patch methodology; a small steps to big results methodology; a commit to healthy eating methodology; and a matched methodology. Preselection may be various modes, e.g., manual selection by a participant, automated selection, a combination thereof, etc.

The medmatch methodology, for example, may incorporate or otherwise be associated with direct or indirect support of an individual or cause in need, an economic consequence, etc. In this example, the donor using the medmatch methodology may empathize to the individual or cause in need to be motivated to make and maintain a behavior change having a positive impact on a health-related outcome for the donor. Similarly, the donor may identify with enabling or avoiding an economic consequence to the donor, a donation recipient, or other(s) to a degree that motivates such a behavior change.

The medmatch methodology may include, but does not necessarily, a verifiable donation transaction, e.g., computer-generated feedback to the donor and other interested parties; a quantifiable donation, e.g., two pills donated for every pill ingested by the donor, etc.

### Article

In various aspects, an article may comprise a non-transitory storage medium having instructions, that when executed by a computing platform, result in execution of a method of communicating health promotion data via a network, comprising / consisting of: receiving, via a hub, the health promotion data; communicating, via the hub, at least a portion of the health promotion data to a methodology module; identifying, via a methodology module, at least one methodology associated with the health promotion data; and generating, via an instruction module, at least one instruction associated with the identified methodology.

The article may further consist / comprise one or more of the following steps of: tracking, via a component of the network, data associated with the health promotion data; generating, via a component of the network, data associated with the health promotion data; and generating, via a component of the network, a preventative action instruction associated with the health promotion data.

Any of the aspects disclosed herein may be performed in a data processing system or by a data processing method, e.g., instructional steps carried out by a computer, processor, etc. To illustrate, a diagrammatic system comprises, for example, a processor, a main memory, a static memory, a bus, a video display, an alpha-numeric input device, a cursor control device, a drive unit, a signal generation device, a network interface device, a machine readable medium, instructions and a network, according to one embodiment.

The diagrammatic system may indicate a personal computer and / or a data processing system in which one or more operations disclosed herein may be performed. The processor may be a microprocessor, a state machine, an application- specific integrated circuit, a field programmable gate array, etc. The main memory may be a dynamic random access memory and / or a primary memory of a computer system. The static memory may be a hard drive, a flash drive, and / or other memory information associated with the data processing system.

The bus may be an interconnection between various circuits and / or structures of the data processing system. The video display may provide graphical representation of information on the data processing system. The alpha-numeric input device may be a keypad, a keyboard and / or any other input device of text, e.g., a special device to aid the physically challenged. The cursor control device may be a pointing device such as a mouse. The drive unit may be a hard drive, a storage system, and / or other longer term storage subsystem. The signal generation device may be a bios and / or a functional operating system of the data processing system. The network interface device may be a device that may perform interface functions such as code conversion, protocol conversion and / or buffering required for communication to and from the network. The machine readable medium may provide instructions on which any of the methods disclosed herein may be performed. The instructions may provide source code and / or data code to the processor to enable any one / or more operations disclosed herein.

Although the present embodiments have been described with reference to specific example embodiments, it will be evident that various modifications and changes may be made to these embodiments without departing from the broader spirit and scope of the various embodiments. For example, the various devices, modules, etc. described herein may be enabled and operated using hardware circuitry, e.g., CMOS based logic circuitry, firmware, software and / or any combination of hardware, firmware, and / or software, e.g., embodied in a machine readable medium.

For example, the various electrical structure and methods may be embodied using transistors, logic gates, and electrical circuits, e.g., Application Specific Integrated circuitry (ASIC) and / or in Digital Signal Processor (DSP) circuitry. For example, the receive module and the communicate module and other modules may be enabled using one or more of the technologies described herein.

In addition, it will be appreciated that the various operations, processes, and methods disclosed herein may be embodied in a machine-readable medium and/or a machine accessible medium compatible with a data processing system, e.g., a computer system, and may be performed in any order. Accordingly, the specification and drawings are to be regarded in an illustrative rather than a restrictive sense.

Any or all data associated with the aforementioned devices and methods, for example, may be used alone or in combination with other data to constitute health promotion data, i.e., data having a health promotion aspect.

In certain embodiments, the system and / or method steps further includes / utilizes an element for storing data, i.e., a data storage element, where this element is present on an external device, such as a bedside monitor, PDA, smart phone, computer server, etc. Typically, the data storage element is a computer readable medium. The term "computer readable medium" as used herein refers to any storage or transmission medium that participates in providing instructions and / or data to a computer for execution and / or processing. Examples of storage media include floppy disks, magnetic tape, CD-ROM, a hard disk drive, a ROM or integrated circuit, a magneto-optical disk, or a computer readable card such as a PCMCIA card and the like, whether or not such devices are internal or external to the computer. A file containing information may be "stored" on a computer readable medium, where "storing" means recording information such that it is accessible and retrievable at a later data by a computer and / or computer-related component. With respect to computer readable media, "permanent memory" refers to memory that is permanent. Permanent memory is not erased by termination of the electrical supply to a computer of processor. Computer hard-drive ROM, i.e., not used as virtual memory, CD-ROM, floppy disk and DVD are all examples of permanent memory. Random Access Memory (RAM) is an example of non-permanent memory. A file in permanent memory may be editable and re-writable.

Aspects extend to any manner of reading and monitoring the sensed parameters of medication and non-medication taking, administration and/or delivery, e.g., an adhesive sensor patch, other wearable sensors, device implants and insertables, parenteral medication delivery devices, mobile phones, etc., and devices to display and manage such information (computers, mobile phones, etc).

Further, various aspects may include one or more data fusion functions. As used herein, the term "data fusion" refers to a process, function, occurrence, event, etc. of data integration, e.g., combining of data, coupled with a reduction, replacement, analysis or other such data manipulation or change that brings about an improved result with respect to the combined data. Examples of improved results include combination of health promotion data and data resulting from one or more of the previously described methodologies from which, when analyzed, a reasonable inference may be drawn that an individual associated with the health promotion data has changed behavior patterns, resulting in improved adherence to a medication regimen and an improved treatment outcome.

Also provided are computer executable instructions, i.e., programming, for performing the above methods, e.g., for programming the IEM, receiver, and other components of the system. The computer executable instructions are present on a computer readable medium. Accordingly, various aspects provide a computer readable medium containing programming for use in providing ingestible event marker data.

As such, in certain embodiments the systems include one or more of: a data storage element, a data processing element, a data display element, a data transmission element, a notification mechanism, and a user interface. These elements may be present or otherwise associated with at least one of the ingestible event marker data, the hub, and the IEM data systems.

One of the above-described systems is reviewed in terms of a receive module and a communicate module. The aspects, however, are not so limited. In a broader sense, the systems are composed of two or more different modules that communicate with each other, e.g., using the hub functionalities as reviewed above, e.g., using the IEM data in the communication, e.g., using the IEM data systems' functionalities.

It is to be understood that this invention is not limited to particular embodiments described, and as such may vary. It is also to be understood that the terminology used herein is for the purpose of describing particular embodiments only, and is not intended to be limiting, since the scope of the present invention will be limited only by the appended claims.

Notwithstanding the claims, the invention is also described by the following clauses:
1. A method consisting of the steps of:
   receiving, by a processor, health promotion data;
   processing, by the processor, the health promotion data to identify at least one preselected behavior change methodology; and
   generating, by the processor, a corresponding instruction to initiate the identified at least one behavior change methodology.
2. The method of clause 1, wherein the health promotion data is generated from at least one event selected from group consisting essentially of an ingestion event, an injection event, an inhalation event, an infusion event, a health monitoring event, a physical activity event, and an eating event, preferably wherein the ingestion event generating the health promotion data is facilitated via an ingestible device, more preferably wherein the ingestible device is selected from a group consisting of an RFID-enabled device and a current-altering device.
3. The method according to any of the preceding clauses wherein the at least one behavior change methodology is selected from a group consisting essentially of a medmatch methodology; a races within reach methodology; a pick a desktop widget / avatar methodology; a family responsibility methodology; a virtual mansion methodology; a daily hatch methodology; a fitimals methodology; a gamefit methodology; a delightful comparators methodology; an adhere to win methodology; a shame game methodology; a pledge matching methodology; a help from my friends methodology; a love buzz methodology; a patch alerts methodology; a patch communicator methodology; a done! buzz methodology; a plug methodology; a real patient profiles methodology; a real futures methodology; a mood miner methodology; a heart fit methodology; a swimmer patch methodology; a small steps to big results methodology; a commit to healthy eating methodology; a placebo pills methodology; and a matched methodology.
4. The method of clause 3, wherein the medmatch methodology is associated with at least one of direct or indirect support of an individual or cause in need and an economic consequence and/or wherein the medmatch methodology is associated with at least one of a verifiable donation transaction and a quantifiable donation transaction.
5. The method according to any of the preceding clauses further consisting of an initial step of generating, by a health-promotion device, the health promotion data, preferably wherein the health-promotion device is an ingestible device.
6. The method according to any of the preceding clauses further consisting of a step of receiving, by a device, the corresponding instruction.
7. The method according to any of the preceding clauses further consisting of at least one step of the steps of:
   tracking, via a system component, data associated with the health promotion data; and
   generating feedback, via a system component, associated with the health promotion data.
8. The method according to any of the preceding clauses further consisting of a step of generating, via a system component, a preventative action instruction associated with the health promotion data.
9. A system, optionally for use in a method according to any of the preceding clauses, the system comprising:
   - health promotion data generated by a device;
   - a methodology module associated with a processor to identify at least one behavior change methodology associated with the health promotion data; and
   - an instruction module associated with the processor to initiate the identified at least one behavior change methodology.
10. The system of clause 9, further comprising a data device to at least one of generate the health promotion data and communicate the health promotion data, wherein the data device is preferably selected from a group consisting essentially of an ingestible device, an injection device, an inhalation device, an infusion device, a detector device, a health monitoring device, a physical activity device, an implantable device, a drug depot release device, and an eating device.
11. The system of clause 10, wherein the ingestible device encodes the health promotion data in a current flow.
12. The system according to clauses 10 or 11 wherein the ingestible device comprises:
   a control device for altering conductance; and
   a partial power source comprising:
      a first material electrically coupled to the control device; and
      a second material electrically coupled to the control device and electrically isolated from the first material;
      wherein the first and second materials are selected to provide a voltage potential difference when in contact with a conducting liquid, and
      wherein the control device alters the conductance between the first and second materials such that the magnitude of the current flow is varied to encode the health promotion data.
13. The system of according to any of the clauses 10-12 wherein the detector device communicates at least one of medication delivery event data and physiologic parameter data.
14. The system according to any of the preceding clauses 9-13 further comprising a tracking / feedback module to at least one of track data associated with the health promotion data and generate feedback associated with the health promotion data.
15. The system according to any of the preceding clauses 9-14 further comprising a preventative action module to generate a preventative action instruction associated with the health promotion data.
16. An article comprising:
   a non-transitory storage medium having instructions, that when executed by a computing platform, result in execution of a method of communicating health promotion data via a network, comprising:
      receiving, via a hub, the health promotion data;
      communicating, via the hub, at least a portion of the health promotion data to a methodology module;
      identifying, via a methodology module, at least one methodology associated with the health promotion data; and
      generating, via an instruction module, at least one instruction associated with the identified methodology.
17. The article of clause 16, further comprising at least one of the following steps of:
   tracking, via a component of the network, data associated with the health promotion data; and
   generating, via a component of the network, data associated with the health promotion data.
18. The article of clause 16 or 17 further comprising a step of generating, via a component of the network, a preventative action instruction associated with the health promotion data.
19. System according to any of the preceding clauses 9-15 or an article according to any of the preceding clauses 16-18 wherein fusion of the health promotion data and data associated with the behavior change methodology directly or indirectly prompt a behavior change.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

All applications, publications, and patents cited in this specification are herein incorporated by reference as if each individual publication or patent were specifically and individually indicated to be incorporated by reference and are incorporated herein by reference to disclose and describe the systems, methods and / or materials in connection with which they are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

Accordingly, the preceding merely illustrates the principles of the invention. It will be appreciated that those skilled in the art will be able to devise various arrangements which, although not explicitly described or shown herein, embody the principles of the invention and are included within its spirit and scope. This application is a divisional application of European patent application no. EP 12734126.1 (the "parent application"), also published under no. EP-A-2663962.

The original claims of the parent application are repeated below in the present specification and form part of the content of this divisional application as filed.

Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of present invention is embodied by the appended claims.
1. A method consisting of the steps of:
   receiving, by a processor, health promotion data;
   processing, by the processor, the health promotion data to identify at least one preselected behavior change methodology; and
   generating, by the processor, a corresponding instruction to initiate the identified at least one behavior change methodology.
2. The method of claim 1, wherein the health promotion data are generated from at least one event selected from group consisting essentially of an ingestion event, an injection event, an inhalation event, an infusion event, a health monitoring event, a physical activity event, and an eating event.
3. The method of claim 2, wherein the ingestion event generating the health promotion data is facilitated via an ingestible device.
4. The method of claim 3, wherein the ingestible device is selected from a group consisting of an RFID-enabled device and a current-altering device.
5. The method of claim 1, wherein the at least one behavior change methodology is selected from a group consisting essentially of a medmatch methodology; a races within reach methodology; a pick a desktop widget / avatar methodology; a family responsibility methodology; a virtual mansion methodology; a daily hatch methodology; a fitimals methodology; a gamefit methodology; a delightful comparators methodology; an adhere to win methodology; a shame game methodology; a pledge matching methodology; a help from my friends methodology; a love buzz methodology; a patch alerts methodology; a patch communicator methodology; a done! buzz methodology; a plug methodology; a real patient profiles methodology; a real futures methodology; a mood miner methodology; a heart fit methodology; a swimmer patch methodology; a small steps to big results methodology; a commit to healthy eating methodology; a placebo pills methodology; and a matched methodology.
6. The method of claim 5, wherein the medmatch methodology is associated with at least one of direct or indirect support of an individual or cause in need and an economic consequence.
7. The method of claim 5, wherein the medmatch methodology is associated with at least one of a verifiable donation transaction and a quantifiable donation transaction.
8. The method of claim 1, further consisting of an initial step of generating, by a health-promotion device, the health promotion data.
9. The method of claim 8, wherein the health-promotion device is an ingestible device.
10. The method of claim 1, further consisting of a step of receiving, by a device, the corresponding instruction.
11. The method of claim 1, further consisting of at least one step of the steps of:
   tracking, via a system component, data associated with the health promotion data; and
   generating feedback, via a system component, associated with the health promotion data.
12. The method of claim 1, further consisting of a step of generating, via a system component, a preventative action instruction associated with the health promotion data.
13. A system, comprising:
   health promotion data generated by a device;
   a methodology module associated with a processor to identify at least one behavior change methodology associated with the health promotion data; and
   an instruction module associated with the processor to initiate the identified at least one behavior change methodology.
14. The system of claim 13, further comprising a data device to at least one of generate the health promotion data and communicate the health promotion data.
15. The system of claim 14, wherein the data device is selected from a group consisting essentially of an ingestible device, an injection device, an inhalation device, an infusion device, a detector device, a health monitoring device, a physical activity device, an implantable device, a drug depot release device, and an eating device.
16. The system of claim 15, wherein the ingestible device encodes the health promotion data in a current flow.
17. The system of claim 16, wherein the ingestible device comprises:
   a control device for altering conductance; and
   a partial power source comprising:
      a first material electrically coupled to the control device; and
      a second material electrically coupled to the control device and electrically isolated from the first material;
      wherein the first and second materials are selected to provide a voltage potential difference when in contact with a conducting liquid, and
      wherein the control device alters the conductance between the first and second materials such that the magnitude of the current flow is varied to encode the health promotion data.
18. The system of claim 15, wherein the detector device communicates at least one of medication delivery event data and physiologic parameter data.
19. The system of claim 13, further comprising a tracking / feedback module to at least one of track data associated with the health promotion data and generate feedback associated with the health promotion data.
20. The system of claim 13, further comprising a preventative action module to generate a preventative action instruction associated with the health promotion data.
21. An article comprising:
   a non-transitory storage medium having instructions, that when executed by a computing platform, result in execution of a method of communicating health promotion data via a network, comprising:
      receiving, via a hub, the health promotion data;
      communicating, via the hub, at least a portion of the health promotion data to a methodology module;
      identifying, via a methodology module, at least one methodology associated with the health promotion data; and
      generating, via an instruction module, at least one instruction associated with the identified methodology.
22. The article of claim 21, further comprising at least one of the following steps of:
   tracking, via a component of the network, data associated with the health promotion data; and
   generating, via a component of the network, data associated with the health promotion data.
23. The article of claim 21, further comprising a step of generating, via a component of the network, a preventative action instruction associated with the health promotion data.
24. A system, comprising:
   health promotion data generated by a device;
   a methodology module associated with a processor to identify at least one behavior change methodology associated with the health promotion data; and
   an instruction module associated with the processor to initiate the identified at least one behavior change methodology,
   wherein fusion of the health promotion data and data associated with the behavior change methodology directly or indirectly prompt a behavior change.

## Claims

1. A system, comprising:
a first server (202) configured to receive first health promotion data and second health promotion data, wherein the first health promotion data (102) comprises an identification of a first individual (200) and at least one of medication delivery event data communicated from a first ingestible device, sleep data collected by a first detector device, or exercise data collected by the first detector device, wherein the second health promotion data comprises an identification of a second individual and at least one of medication delivery event data communicated from a second ingestible device, sleep data collected by a second detector device, or exercise data collected by the second detector device, and wherein the first server comprises:
a methodology module (104) configured to:
identify a first behavior change program associated with the first health promotion data based on the identification of the first individual (200); and
identify a second behavior change program associated with the second health promotion data based on the identification of the second individual; and
an instruction module (106) configured to:
initiate a software application, wherein the software application administers the first behavior change program and the second behavior change program; and
a second server (204) communicatively coupled to the first server (202), wherein the second server (204) executes the software application configured to:
analyze the first health promotion data to determine an adherence of the first individual (200) to at least one of a medication regimen, a sleep schedule, or an exercise program associated with the first behavior change program;
analyze the second health promotion data to determine an adherence of the second individual to at least one of a medication regimen, a sleep schedule, or an exercise program associated with the second behavior change program;
provide a first virtual representation of a status of the first individual and update the first virtual representation based on the adherence of the first individual to the at least one of the medication regimen, the sleep schedule, or the exercise program associated with the first behavior change program;
provide a second virtual representation of a status of the second individual and update the second virtual representation based on the adherence of the second individual to the at least one of the medication regimen, the sleep schedule, or the exercise program associated with the second behavior change program; and
display, when the first individual and the second individual are members of the same family, the updated first virtual representation and the updated second virtual representation on a common computer user interface.

2. The system of claim 1, further comprising:
a first on-body communication patch configured to receive the medication delivery event data communicated from the first ingestible device and forward the first health promotion data to a first mobile device for onward communication to the first server; and
a second on-body communication patch configured to receive the medication delivery event data communicated from the second ingestible device and forward the second health promotion data to a second mobile device for onward communication to the first server.

3. The system of claim 1 or claim 2, wherein each of the first ingestible device and the second ingestible device is selected from a group consisting of an RFID-enabled device and a current-altering device.

4. The system of any of claims 1 to 3, wherein updating the first virtual representation comprises visually morphing the first virtual representation as the status of the first individual changes, and wherein updating the second virtual representation comprises visually morphing the second virtual representation as the status of the second individual changes.

5. The system of any of claims 1 to 4, wherein the first virtual representation and the second virtual representation are linked by a common theme.

6. The system of any of claims 1 to 5, wherein the first virtual representation is an avatar selected by the first individual and the second virtual representation is an avatar selected by the second individual.

7. The system of claim 1, wherein the first ingestible device comprises:
a control device for altering conductance; and
a partial power source comprising:
a first material electrically coupled to the control device; and
a second material electrically coupled to the control device and electrically isolated from the first material;
wherein the first material and the second material are selected to provide a voltage potential difference when in contact with a conducting liquid; and
wherein the control device alters the conductance between the first material and the second material such that the magnitude of the current flow is varied to encode the first health promotion data in the current flow.

8. The system of claim 1, wherein the second ingestible device comprises:
a control device for altering conductance; and
a partial power source comprising:
a first material electrically coupled to the control device; and
a second material electrically coupled to the control device and electrically isolated from the first material;
wherein the first material and the second material are selected to provide a voltage potential difference when in contact with a conducting liquid; and
wherein the control device alters the conductance between the first material and the second material such that the magnitude of the current flow is varied to encode the second health promotion data in the current flow.
